# FASCICULE DE BREVET EUROPEEN

(11) **EP 0 669 322 B1**
(45) Date de publication et mention de la délivrance du brevet: **15.09.1999**
(21) Numéro de dépôt: 95400384.4
(22) Date de dépôt: 23.02.1995
(51) Int. Cl.: C07D 217/02, C07D 271/12, C07D 311/14, C07D 285/14, C07D 295/12, C07D 401/12, C07D 213/42

(54) **Sulfonamides substituées, leur procédé de préparation et les compositions pharmaceutiques les renfermant**
Substituierte Sulfonamide, Verfahren zu deren Herstellung und sie enthaltende pharmazeutische Zusammensetzungen
Substituted sulfonamides, process for their preparation and pharmaceutical compositions containing them

(30) Priorité: 25.02.1994 FR 9402159
(43) Date de publication de la demande: 30.08.1995
(73) Titulaire: ADIR ET COMPAGNIE, 92415 Courbevoie Cédex (FR)
(72) Inventeur: Peglion, Jean-Louis, F-78110 Le Vesinet (FR); Vilaine, Jean-Paul, F-92290 Chatenay Malabry (FR); Villeneuve, Nicole, F-92500 Rueil Malmaison (FR); Iliou, Jean-Pierre, F-92800 Puteaux (FR); Bidouard, Jean-Pierre, F-91380 Chilly Mazarin (FR)
(74) Mandataire: Reverbori, Marcelle

(56) Documents cités:
- EP-A- 0 330 065
- EP-A- 0 431 944
- EP-A- 0 525 203
- US-A- 3 527 768

## Description

La présente invention a pour objet de nouvelles sulfonamides substituées, leur procédé de préparation et les compositions pharmaceutiques les renfermant.

Elle concerne plus particulièrement :
- les sulfonamides substituées de formule I : 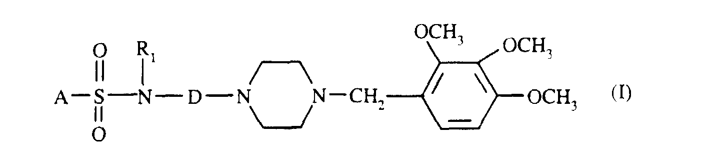 dans laquelle :
   - A représente un radical de formule : 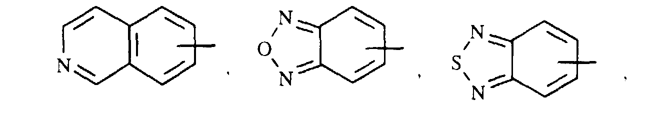 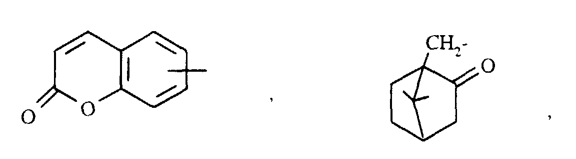 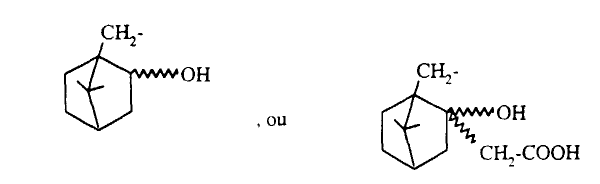
   - R₁ représente :
      a) un atome d'hydrogène,
      b) un radical alkyle linéaire de 1 à 7 atomes de carbone éventuellement substitué par un ou plusieurs radicaux méthyle, phényle, pyridyle ou thiényle, eux-mêmes éventuellement substitués soit par un ou plusieurs atomes d'halogène, soit par un radical hydroxy,
      c) un radical alkényle de 3 à 7 atomes de carbone (comme par exemple -CH₂-CH=CH₂), ou
      d) un radical alkynyle de 3 à 7 atomes de carbone (comme par exemple

         -CH₂-C≡CH ) ;

         et
   - D représente :
      a) une chaîne hydrocarbonée linéaire saturée contenant de 2 à 6 atomes de carbone interrompue par un ou plusieurs atomes d'oxygène, de soufre ou un cycle cyclopropane ou substituée par un gem-diméthyle, ou
      b) un groupement de formule : 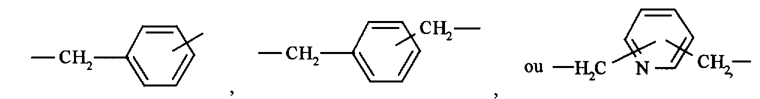
- ainsi que leurs sels d'addition physiologiquement tolérables avec des acides appropriés.

L'état antérieur de la technique le plus proche de la présente invention est illustré par la demande de brevet européen, oubliée sous le n° 0 330 065 A, qui conceme des sulfonamides de formule :

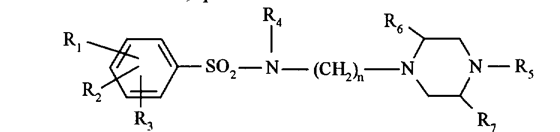

c'est à dire des composés dans lesquels la fonction sulfonamide est toujours liée à un cycle benzénique lui-même éventuellement substitué mais qui n'inclut ni ne suggère nullement les radicaux bicycliques de formule A tels que définis pour la formule I précédemment décrite.
On peut également citer les demandes de brevets :
- EP 0 431 944 qui concerne des agents antiarhytmiques de classe III de formule : 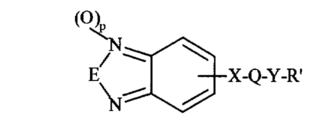
dans laquelle :
- E peut être oxygène ou soufre, - X : SO₂, - Q: 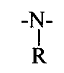 Y : (CH₂)ₙ' et
- R' : hydrogène ou un cycle aromatique ;
et
- EP 0 525 203 qui concerne des produits bronchodilatateurs de formule : 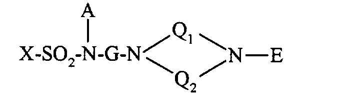 qui ne suggèrent pas davantage les composés de la présente demande.

La présente invention a aussi pour objet le procédé de préparation des composés de formule I caractérisé en ce que l'on fait réagir un sulfochlorure de formule II :

A-SO₂ Cl (II)

dans laquelle A prend la signification précédemment définie,
avec une amine de formule III : 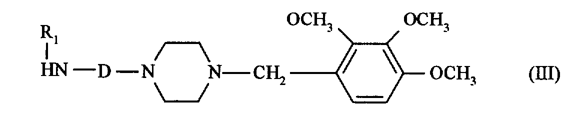 dans laquelle R₁ et D ont les significations précédemment définies.

D'autre part, les composés de formule I dans laquelle R₁ prend les valeurs énoncées précédemment à l'exception de la valeur hydrogène, c'est à dire les composés répondant à la formule I' : 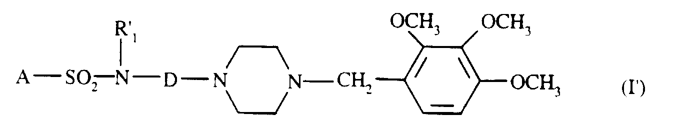 dans laquelle :
A et D ont les significations précédemment définies et R'₁ représente :
α) un radical alkyle linéaire de 1 à 7 atomes de carbone éventuellement substitué par un ou plusieurs radicaux méthyle, phényle pyridyle ou thiényle, eux-mêmes éventuellement substitués soit par un ou plusieurs atomes d'halogène, soit par un radical hydroxy,
β) un radical alkényle de 3 à 7 atomes de carbone (comme par exemple -CH₂-CH=CH₂), ou
χ) un radical alkynyle de 3 à 7 atomes de carbone (comme par exemple -CH₂-C≡CH )
peuvent également être préparés en faisant réagir les composés de formule I dans laquelle R₁ est uniquement un atome d'hydrogène, c'est à dire en faisant réagir les composés de formule I'' : 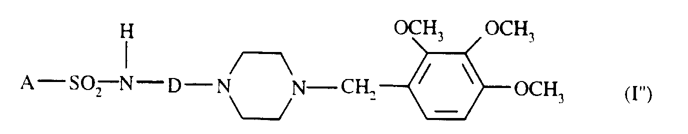 dans laquelle A et D ont les significations précédemment définies,
avec de l'hydrure de sodium puis un halogénure de formule II' :

R'₁ X (II')

dans laquelle R'₁ prend la signification énoncée précédemmment et X représente un atome d'halogène, en opérant dans un solvant approprié tel que le N,N-diméthylacétamide.

Les composés de formule I peuvent être transformés en sels d'addition avec des acides physiologiquement tolérables, sels qui font, à ce titre, partie de l'invention. Comme acides utilisables pour la formation de ces sels, on peut citer, par exemple, dans la série minérale, les acides chlorhydrique, bromhydrique, nitrique, sulfurique, phosphorique et dans la série organique, les acides acétique, propionique, maléique, fumarique, tartrique, oxalique, benzoïque, méthanesulfonique et iséthionique.

Tous les sulfochlorures de départ de formule II sont décrits dans la littérature.
Les matières premières de formule III ont été préparées à partir de produits connus par des méthodes diverses selon les significations de D et R₁ comme mentionné dans les exemples ci-après.

Les composés de la présente invention possèdent des propriétés pharmacologiques et thérapeutiques intéressantes. En particulier pour ces composés, ont été mis en évidence :
. **IN VITRO**
   - d'une part : leur activité antihypoxique prévenant la dysfonction de coeurs isolés de rat au cours de protocoles d'hypoxie réoxygénation et limitant la nécrose de cellules cardiaques induite par une hypoxie
   - d'autre part: leur capacité protectrice vis-à-vis d'un modèle de surcharge calcique intra-cellulaire : la nécrose de cellules cardiaques de rat induite par paradoxe du calcium ;
   et
. **IN VIVO :** leur activité anti-ischémique au cours de protocoles d'ischémie myocardique induite par sténose coronaire chez le porc.

Ces propriétés permettent l'utilisation des composés de la présente invention comme médicament dans le traitement préventif ou curatif des pathologies ischémiques notamment dans le domaine cardiovasculaire : angine de poitrine, infarctus du myocarde et les conséquences des cardiopathies ischémiques (trouble du rythme, insuffisance cardiaque) et de la pathologie vasculaire périphérique.
Les dérivés de la présente invention peuvent également être utilisés dans le domaine cérébral, notamment pour le traitement de l'accident vasculaire cérébral et des manifestations déficitaires liés aux troubles circulatoires cérébraux chroniques ; dans le domaine ophtalmologique : notamment pour le traitement des troubles rétiniens d'origine vasculaire ; et dans les manifestations neurosensorielles d'origine ischémique.

La posologie peut varier notablement selon l'âge et le poids du patient, la voie d'administration, la nature de la maladie et les traitements associés et s'échelonne de 1 à 200 mg de principe actif, 1 à 3 fois par jour.

La présente invention a également pour objet les compositions pharmaceutiques contenant comme principe actif un composé de formule I ou un de ses sels physiologiquement tolérable, mélangé ou associé à un ou plusieurs excipients pharmaceutiques appropriés.

Les compositions pharmaceutiques ainsi obtenues se présentent généralement sous forme dosée renfermant de 1 à 200 mg de principe actif. Elles peuvent par exemple revêtir la forme de comprimés, dragées, gélules, suppositoires, solutions injectables ou buvables et être administrées selon les cas par voie orale, rectale ou parentérale.

Les exemples suivants illustrent la présente invention. Les points de fusion sont, sauf mention contraire, déterminés à la platine chauffante de Kofler.

### Exemple 1

### N-éthyl N-{3,3-diméthyl 4-[4-(2,3,4-triméthoxybenzyl)pipérazin- 1-yl] butyl} (isoquinoléin-5-yl)sulfonamide :

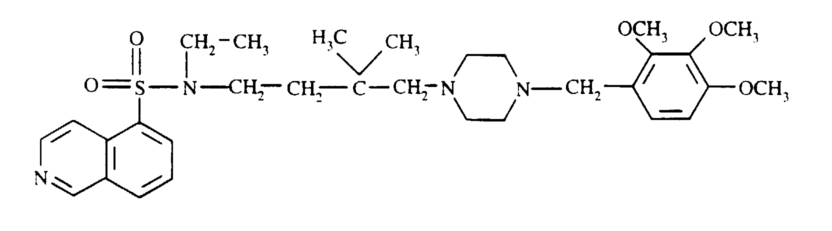

1) On ajoute goutte à goutte 0,1 mole de chlorure de l'acide 3-chloro 2,2-diméthyl propionique sous agitation au mélange de 0,1 mole de N-(2,3,4-triméthoxybenzyl)pipérazine, 0,1 mole de triéthylamine dans 250 ml de benzène. Après une nuit de contact sous agitation, on transvase en ampoule et lave à l'eau. On sépare le produit attendu de formule : 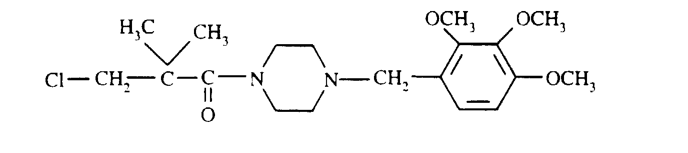 sous forme d'huile avec un rendement de 54 %.
2) 0,053 mole du produit ainsi obtenu et 0,063 mole d'iodure de sodium dans 600 ml de méthyléthylcétone sont portés à reflux pendant 24 heures. Après contrôle par HPLC, on rajoute 0,063 mole d'iodure de sodium suivi d'un reflux de 24 heures. On ramène l'ensemble à température ambiante, évapore, reprend à l'éther, lave au thiosulfate de sodium normal. On obtient le produit attendu de formule : 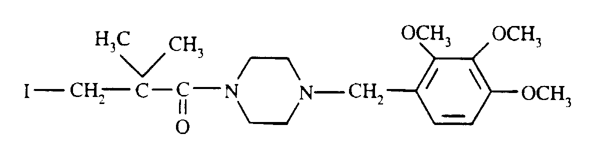 sous forme d'huile avec un rendement de 95 %.
3) Le dérivé iodé ci-dessus obtenu est porté à 100 °C sous agitation avec deux équivalents de cyanure de sodium dans 100 ml de diméthylformamide pendant 6 heures. On évapore ensuite le diméthylformamide, reprend à l'eau et extrait à l'éther. Le nitrile ainsi obtenu, sous forme d'huile, avec un rendement de 54 % est utilisé tel quel sans autre purification.
4) Le nitrile obtenu ci-dessus est dissout dans 250 ml de tétrahydrofurane et la solution ainsi obtenue est soumise à l'action de 10 équivalents de borane diméthyl sulfure. Le mélange réactionnel est porté au reflux pendant 6 heures.
   Après solvolyse avec 25 ml de méthanol puis évaporation et hydrolyse par 40 ml d'HCl concentré dans 80 ml de méthanol, on évapore, reprend à l'eau, extrait à l'éther, basifie la phase aqueuse et extrait à l'acétate d'éthyle. On obtient l'amine attendue sous forme d'huile, avec un rendement de 90 %.
5) A 0,017 mole de l'amine ci-dessus obtenue et 0,034 mole de triethylamine dans 65 ml de chlorure de méthylène, on ajoute par fractions, à température ambiante, 0,017 mole de chlorhydrate d'(isoquinolein-5-yl) sulfochlorure. On laisse en contact sous agitation durant la nuit, puis transvase en ampoule, lave par 100 ml de soude normale et sèche la phase organique. Ensuite, on chromatographie dans un système CH₂Cl₂-CH₃OH (95-5) sur colonne de silice et recueille 6,3 g de produit attendu, sous forme d'huile
6) 2,8 g (0,005 mole) du sulfonamide préparé à l'étape précédente, dissout dans 30 ml de diméthyl acétamide, sont sodés avec la quantité stoechiométrique d'hydrure de sodium à 60 %. Après la fin du dégagement gazeux, on ajoute 0,005 mole d'iodure d'éthyle et laisse le mélange réactionnel sous agitation durant la nuit. Puis on dilue abondamment à l'eau et extrait à l'acétate d'éthyle, sèche et évapore. On obtient alors le produit titre de l'exemple 1, sous forme d'huile avec un rendement de 64 %. Le difumarate du N-éthyl N-{3,3-diméthyl 4-[4-(2,3 ,4-triméthoxybenzyl) pipérazin-1-yl] butyl} (isoquinoléin-5-yl)sulfonamide fond à 143-146 °C.

### Exemples 2 - 5

En opérant par analogie avec la méthode décrite dans l'exemple 1, ont été préparés les composés objet des exemples suivants :
2) Le N-benzyl N-{3,3-diméthyl 4-[4-(2,3,4-triméthoxybenzyl) pipérazin-1-yl]butyl} (isoquinoléin-5-yl)sulfonamide, PF du 2,5 fumarate correspondant : 174-176 °C.
3) Le (1R) N-éthyl N-{3,3-diméthyl 4-[4-(2,3,4-triméthoxybenzyl) pipérazin-1-yl]butyl}
   10-camphosulfonamide, PF du dichlorhydrate correspondant : 243-245 °C.
4) Le (1S) N-éthyl N-{3,3-diméthyl 4-[4-(2,3,4-triméthoxybenzyl)pipérazin-1-yl]butyl}
   10-camphosulfonamide, PF du dichlorhydrate correspondant : 243-245 °C.
5) Le (1R) N-benzyl N-{3,3-diméthyl 4-[4-(2,3,4-triméthoxybenzyl)pipérazin-1-yl]butyl} 10-camphosulfonamide, PF du dichlorhydrate correspondant : 188-191 °C.

### Exemple 6

### N-benzyl N-{[4-[4-(2,3,4-triméthoxybenzyl)pipérazin-1-yl]phényl]méthyl} (isoquinoléin -5-yl)sulfonamide

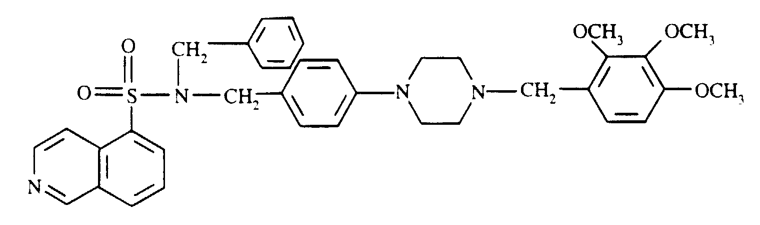

1) On porte à 100 °C sous agitation pendant 8 heures 0,2 mole de fluorobenzonitrile, 0,2 mole de carbonate de potassium et 0,2 mole de N-(2,3,4-triméthoxybenzyl)pipérazine. Puis on dilue à l'eau, extrait à l'éther, épuise la phase organique à l'acide chlorhydrique normal, basifie les phases aqueuses réunies, à froid, par de la soude concentrée et extrait à l'acétate d'éthyle. On obtient le nitrile attendu, sous forme d'huile, avec un rendement de 40 %.
2) Le nitrile ainsi obtenu est réduit par un équivalent d'aluminohydrure de lithium dans 150 ml de tétrahydrofurane. Après décomposition, on obtient une huile que l'on soumet à une chromatoflash en éluant avec CH₂Cl₂/CH₃OH/NH₄OH (95/5/0,5).On obtient l'amine attendue, sous forme d'une huile, avec un rendement de 37 %.
3) Le couplage de l'amine ci-dessus obtenue avec l'(isoquinoléin-5-yl)sulfochlorure est réalisé selon la méthode décrite dans l'exemple 1 paragraphe 5). La N-alkylation est réalisée par la méthode décrite dans l'exemple 1 paragraphe 6) mais en utilisant le bromure de benzyle à la place de l'iodure d'éthyle. On obtient ainsi le produit titre de l'exemple 6 dont le chlorhydrate fond à 152-155 °C.

### Exemple 7 :

### Le cis N-éthyl N-{2-[4-(2,3,4-triméthoxybenzyl)pipérazin-1-yl méthyl]cyclopropylméthyl} (isoquinoléin-5-yl)sulfonamide.

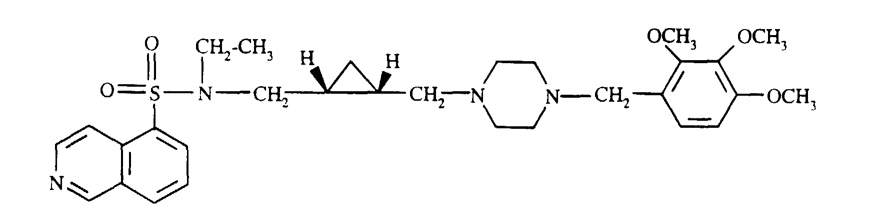

1) Préparation du mélange cis-trans de 2-cyanocycloprop-1-yl carboxylate de méthyle : 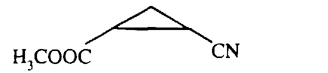
On coule sur une suspension de 50 ml de toluène et 0,5 mole d'hydrure de sodium, le mélange de 0,5 mole d'acrylonitrile et de 0,5 mole de chloroacétate de méthyle. On laisse le mélange réactionnel sous agitation pendant une nuit, puis on décompose très lentement par 150 ml d'éther contenant 16 ml de méthanol. On transvase en ampoule, dilue à l'éther, lave par une solution saturée de chlorure de sodium, sèche et évapore.
   Par distillation on recueille le mélange cis-trans, Eb/_{15 mm} = 120-140 °C (Rendement : 20 %).
2) Préparation du mélange cis-trans d'acide 2-cyanocycloprop-1-yl carboxylique : 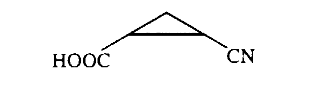
   On saponifie 11,7 g (0,093 mole) du 2-cyanocycloprop-1-yl carboxylate de méthyle précédemment obtenu avec 100 ml de soude normale et 50 ml d'éthanol, sous agitation durant une nuit. On évapore l'alcool, ajoute 100 ml d'acide chlorhydrique normal, évapore à sec à poids constant.
   On reprend le précipité par 100 ml d'acétonitrile, filtre le chlorure de sodium et évapore. On obtient le mélange cis-trans d'acide 2-cyanocycloprop-1-yl carboxylique, PF : 80-90 °C (Rendement : 81 %).
3) Préparation de 1-cyano-{2-[4-(2,3,4-triméthoxybenzyl)pipérazin-1-yl] carbonyl} cyclopropane sous formes séparées d'isomères cis et trans : 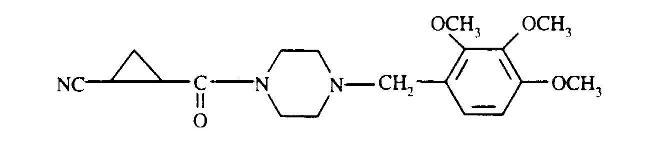
   On ajoute en une fois 12,2 g (0,075 mole) de carbonyl diimidazole à la suspension de 8,4 g (0,075 mole) d'acide 2-cyanocycloprop-1-yl carboxylique et de 50 ml de CH₂Cl₂ et laisse l'ensemble sous agitation durant deux heures après la fin du dégagement gazeux. On coule goutte à goutte 20 g (0,075 mole) de 4-(2,3,4-triméthoxybenzyl)pipérazine dans 100 ml de CH₂Cl₂ et laisse l'ensemble sous agitation durant la nuit. Puis on transvase en ampoule, lave à l'eau, sèche et évapore. Après chromatoflash, on obtient 8,3 g (35 %) de l'isomère trans (huile) et 11,1 g (47 %) de l'isomère cis, PF : 108-110 °C.
4) Préparation de la cis-2-{[4-(2,3,4-triméthoxybenzyl)pipérazin-1-yl] méthyl} cycloprop-1-yl méthylamine. : 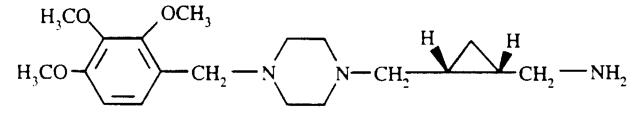
   On coule goutte à goutte 22,7 g (0,3 mole) de borane diméthyl sulfure sur la solution agitée de 8,3 g du cis nitrile préalablement obtenu dans 315 ml de tétrahydrofurane. On porte l'ensemble à reflux durant 6 heures. On ramène à température ambiante, décompose lentement par 20 ml de méthanol et maintient à reflux jusqu'à la fin du dégagement gazeux. Puis on évapore, reprend par 50 ml de méthanol et 10 ml d'acide chlorhydrique concentré et porte à reflux jusqu'à la fin du dégagement gazeux.
   On évapore ensuite le méthanol, basifie à froid la phase aqueuse, extrait à l'éther, sèche et évapore. Rendement : 2,3 g (29 %).
5) Préparation du composé cis de formule : 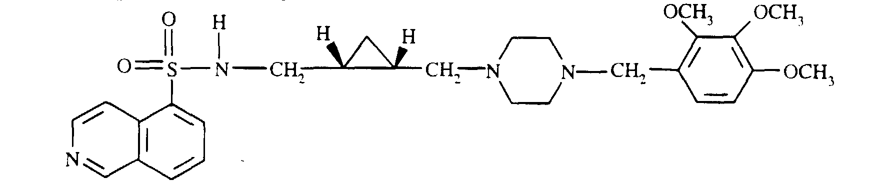
   On ajoute par fractions 1,1 g (0,004 mole) de chlorhydrate d'(isoquinoléin-5-yl)sulfochlorure à une solution de 2,3 g (0,008 mole) de cis-{{2-[4-(2,3,4-triméthoxybenzyl) piperazin-1-yl]méthyl}cycloprop-1-yl} méthylamine et 0,8 g de triéthylamine dans 25 ml de CH₂Cl₂. On laisse en contact durant une nuit puis transvase en ampoule, lave à la soude normale, puis à l'eau et évapore. Après chromatoflash en éluant avec CH₂Cl₂/CH₃OH (95/5), on obtient 1,2 g du produit attendu avec un rendement de 57 %.
6) Préparation du composé titre de l'exemple 7 :
   1,2 g (0,0024 mole) du sulfonamide obtenu en 5) dans 15 ml de diméthylacétamide est sodé avec 0,1 g d'hydrure de sodium à 60 %. Puis on ajoute 0,4 g d'iodure d'éthyle et laisse le mélange sous agitation à température ambiante durant la nuit. On dilue à l'eau et extrait à l'acétate d'éthyle. Après chromatoflash en éluant avec CH₂Cl₂/CH₃OH (95/5), on obtient 1g de substance désirée (Rendement : 80 %). La salification est réalisée par dissolution dans 5 ml d'acétate d'éthyle auxquels on ajoute 3 équivalents d'HCl N dans l'éther.On filtre, sèche, et cristallise le produit dans 5 ml de cyanure de méthyle. On obtient ainsi le trichlorhydrate de cis-N-éthyl N-{**2**-[4-(2,3,4-triméthoxybenzyl)pipérazin-1-yl méthyl] cyclopropylméthyl} (isoquinoléin-5-yl)sulfonamide, PF : 175 °C.

### Exemple 8

### Le trans-N-éthyl N-{2-[4-(2,3,4-triméthoxybenzyl)pipérazin-1-yl méthyl] cyclopropylméthyl} (isoquinoléin-5-yl)sulfonamide :

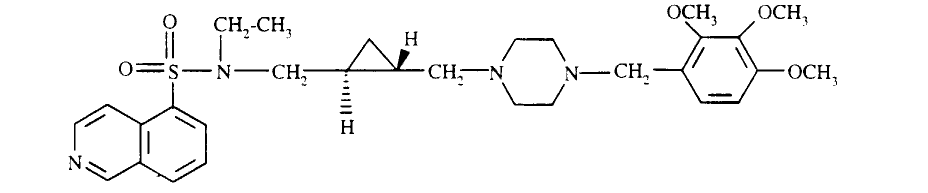

a été préparé par analogie avec la méthode décrite dans l'exemple 7 mais en remplaçant à partir du paragraphe 4) le composé cis par le dérivé trans correspondant. Le trichlorhydrate du produit titre de l'exemple 8 fond à 162-165 °C.

### Exemple 9

### Le cis-N-benzyl N-{2-[4-(2,3,4-triméthoxybenzyl)pipérazin-1-yl méthyl] cyclopropyl méthyl} (isoquinoléin-5-yl)sulfonamide :

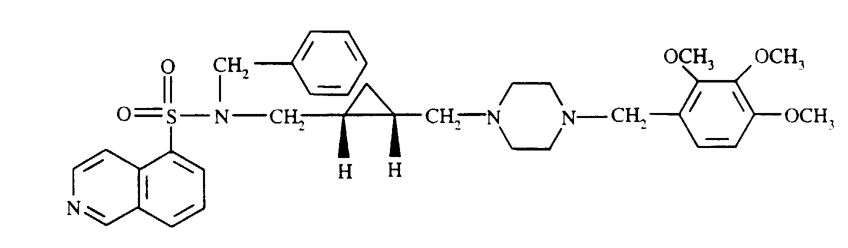

a été préparé comme indiqué dans l'exemple 7 mais en remplaçant dans le paragraphe 6) l'iodure d'éthyle par le bromure de benzyle.
On obtient ainsi le trichlorhydrate de cis-N-benzyl N-{2-[4-(2,3,4-triméthoxybenzyl)pipérazin-1-yl méthyl] cyclopropylméthyl} (isoquinoléin-5-yl)sulfonamide, PF : 202-204 °C.

### Exemple 10

### N-(pyrid-3-yl méthyl) N-{3,3-diméthyl 4-[4-(2,3,4-triméthoxybenzyl)pipérazin-1-yl]butyl} (isoquinoléin-5-yl)sulfonamide :

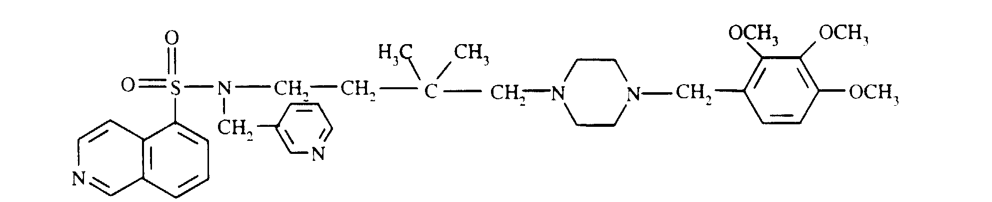

1) Préparation de l'amine de formule : 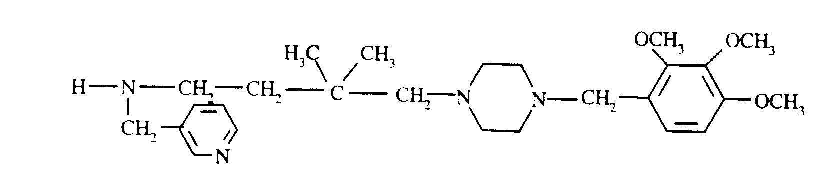
   7,3 g (0,02 mole) de l'amine obtenue au paragraphe 4) de l'exemple 1 sont portés au reflux, dans 80 ml d'éthanol, avec 2,14 g (0,02 mole) de 3-pyridine carbonaldéhyde.
   Après retour à température ambiante, on ajoute par portions, 0,8 g de borohydrure de sodium. On laisse en contact pendant 4 heures après la fin du dégagement gazeux. Puis on dilue à l'eau et extrait à l'éther. Après séchage et évaporation, on obtient 6,5 g d'une huile dont les caractéristiques correspondent au produit attendu et que l'on utilise tel quel, sans autre purification.
2) Préparation du composé titre de l'exemple 10 :
   L'amine ci-dessus obtenue est condensée avec le chlorhydrate d'(isoquinoléin-5-yl)sulfochlorure, selon la méthode décrite dans l'exemple 1 paragraphe 5) pour conduire au composé titre de l'exemple 10 dont le tétrachlorhydrate fond à une température supérieure à 260 °C.

### Exemples 11-14

En opérant selon la méthode décrite dans l'exemple 10, ont été préparés les composés objet des exemples suivants :
11) le N-(pyrid-2-yl méthyl) N-{3,3-diméthyl 4-[4-(2,3,4-triméthoxybenzyl) pipérazin-1-yl] butyl} (isoquinoléin-5-yl)sulfonamide, en remplaçant, au paragraphe 1) de l'exemple 10, le 3-pyridinecarbonaldéhyde par le 2-pyridinecarbonaldéhyde.
   La base libre obtenue fond à 110-113 °C.
12) Le N-(pyrid-4-yl méthyl) N-{3,3-diméthyl 4-[4-(2,3,4-triméthoxybenzyl)pipérazin-1-yl] butyl} (isoquinoléin-5-yl)sulfonamide, en remplaçant, au paragraphe 1) de l'exemple 10, le 3-pyridine carbonaldéhyde par le 4-pyridine carbonaldéhyde. Le tétraméthanesulfonate du produit titre fond à 143-146 °C
13) Le N-(thién-2-yl méthyl) N-{3,3-diméthyl 4-[4-(2,3,4-triméthoxybenzyl)pipérazin-1-yl] butyl} (isoquinoléin-5-yl)sulfonamide, en remplaçant, au paragraphe 1 de l'exemple 10, le 3-pyridinecarbonaldéhyde par le 2-thiophènecarbonaldéhyde. Le trichlorhydrate du produit titre fond à 188-190 °C.
14) Le N-(thién-3-yl méthyl) N-{3,3-diméthyl 4-[4-(2,3,4-triméthoxybenzyl)pipérazin-1-yl] butyl} (isoquinoléin-5-yl)sulfonamide, en remplaçant, au paragraphe 1) de l'exemple 10, le 3-pyridinecarbonaldéhyde par le 3-thiophènecarbonaldéhyde.
   Le trichlorhydrate du produit titre fond à 158-160 °C.

### Exemple 15

### N-éthyl N-{{4-[4-(2,3,4-triméthoxybenzyl)pipérazin-1-yl]méthyl}benzyl} (isoquinoléin-5-yl)sulfonamide :

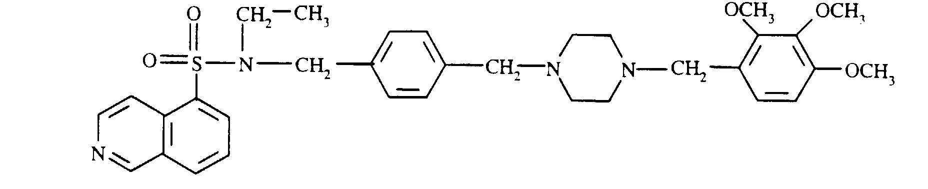

1) Préparation du nitrile de formule : 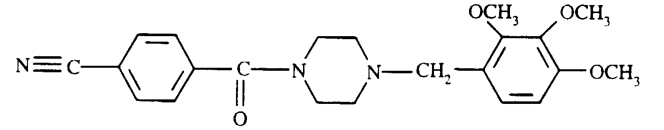
   A une suspension de 25 g (0,17 mole) d'acide p-cyanobenzoique, dans 250 ml de chlorure de méthylène, on ajoute 27,6 g (0,17 mole) de carbonyldiimidazole.
   A la fin du dégagement gazeux, on laisse encore deux heures en contact. A la solution formée, on ajoute alors par un goutte à goutte rapide une solution de 45,3 g (0,17 mole) de 1-(2,3,4-triméthoxybenzyl)pipérazine en solution dans 100 ml de chlorure de méthylène. Après avoir laissé le mélange réactionnel à température ambiante pendant la nuit, on lave à l'eau puis à la soude, puis de nouveau à l'eau. On sèche sur sulfate de magnésium et évapore pour obtenir 65,8 g de produit attendu, sous forme d'huile.
2) Préparation de l'amine de formule : 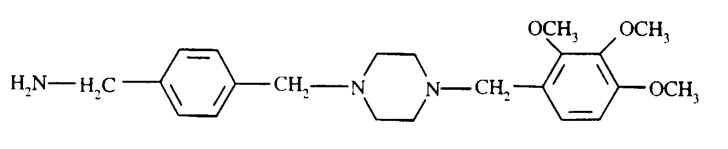
   A 10 g (0,0278 mole) du produit obtenu à l'étape précédente dissout dans 280 ml de tétrahydrofurane, on ajoute goutte à goutte en 15 minutes environ 21 ml de borane diméthyl sulfure.
   On maintient à reflux pendant une nuit. On laisse refroidir puis solvolyse par 43,7 ml de méthanol contenant quelques gouttes d'acide sulfurique. Après un reflux de 4 heures, on évapore à sec, reprend par du chlorure de méthylène, lave à l'eau et sèche sur MgSO₄. On obtient après évaporation 5 g du produit attendu, sous forme d'huile.
3) Préparation du composé de formule : 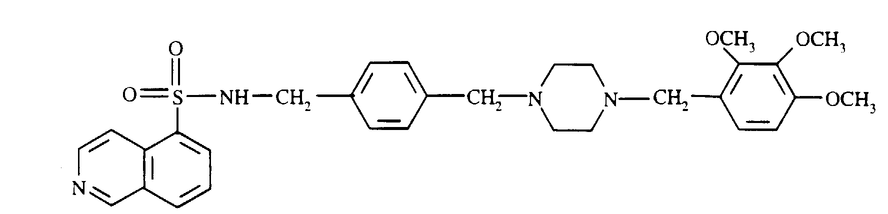
   L'amine ci-dessus obtenue [15 g (0,014 mole)] est condensée avec le chlorhydrate d'(isoquinoléin-5-yl)sulfochlorure selon la méthode décrite dans l'exemple 1 paragraphe 5) pour conduire au composé attendu, après purification par chromatographie sur colonne de silice, en éluant avec CH₂Cl₂/CH₃OH (95/5), et obtenir 2,4 g de composé pur.
4) Préparation du composé titre :
   2,4 g (0,0041 mole) du composé ci-dessus obtenu sont traités selon le mode opératoire décrit dans l'exemple 1 paragraphe 6). On obtient, après purification par chromatographie flash sur silice en éluant avec CH₂Cl₂/CH₃OH (95/5), 2,1 g de base libre qui traitée par 3,5 ml d'éther chlorhydrique 3,5 N conduit au trichlorhydrate du composé titre, PF : 220-222 °C.

### Exemple 16

### ETUDE PHARMACOLOGIQUE

L'activité protectrice cardiaque des produits de la présente invention a été démontrée :
*In vitro*
   . d'une part sur coeurs isolés de rats soumis à un cycle d'hypoxie réoxygénation ainsi que sur des cellules cardiaques de rat soumises à une nécrose hypoxique,
   . d'autre part sur un modèle de surcharge calcique intracellulaire: la nécrose de cellules cardiaques de rat induite par paradoxe du calcium,
   et
*In vivo*
au cours d'épisodes d'ischémie myocardique induite par sténose coronaire chez le porc.
**A - ETUDE IN VITRO**
**1 - MATERIEL ET METHODE**
**1.1. - Hypoxie réoxygénation sur coeur isolé de rat**
   Les coeurs de rats mâles Wistar (325 - 375g - élevage Charles River) anesthésiés par voie intrapéritonéale au pentobarbital sodique (30mg/kg IP) sont prélevés après injection d'héparine IV (1ml/kg) et rapidement perfusés selon la technique de Langendorff à une pression constante de 76mmHg et stimulés électriquement à 5Hz par l'intermédiaire d'électrodes de platine. Les contractions isovolumétriques sont enregistrées par l'intermédiaire d'un ballonnet en polyéthylène relié à un capteur de pression (P23- Gould) introduit dans le ventricule gauche de manière à obtenir une pression diastolique d'environ 10mmHg.
   La solution physiologique utilisée, thermostatée à 37°C, a la composition suivante (mM): NaCl, 118; KCl, 4,7; KH₂PO₄, 1,2; MgCl₂, 1,2; CaCl₂, 1,3; NaHCO₃, 25; Glucose, 8; pH: 7,4;95% O₂+5%CO₂.
   Après une période de stabilisation de 20 à 30 minutes, le coeur est soumis à une hypoxie de 60 minutes (réalisée par 95% de N₂ + 5% CO₂; PO₂<60 mmHg) suivie par une réoxygénation de 30 minutes, le composé à tester est mis en incubation 15 minutes avant et pendant la durée de l'hypoxie. Pour les expériences ex-vivo, le composé est administré par voie orale (1ml/kg) 3 heures avant le sacrifice de l'animal.
**1.2. - Etudes sur cellules cardiaques de rat**
   Les cultures primaires de cardiomyocytes sont réalisées à partir de coeur de rats nouveau-nés. Les cellules cardiaques sont utilisées entre les quatrième et sixième jours après la mise en culture.
   **1.2.1. Nécrose de cellules cardiaques de rat induite par hypoxie.**
      Les cellules soumises à l'hypoxie sont incubées 3 ou 4 heures sous azote dans une chambre de pression thermostatée à 37°C. Les cellules sont traitées avec les molécules testées uniquement au moment de la mise en hypoxie. La nécrose cellulaire induite par l'hypoxie est évaluée en mesurant spectrophotométriquement le pourcentage cellulaire de lactate dehydrogénase libéré dans le surnageant après 3 ou 4 heures d'incubation.
   **1.2.2. Nécrose de cellules cardiaques de rat induite par paradoxe du calcium**
      Les cellules cardiaques sont tout d'abord incubées 30 minutes dans un tampon sans calcium ni magnésium, mais supplémenté avec 1mM en EDTA. Après élimination du surnageant, les cellules sont ensuite incubées 4 heures dans un tampon 3.8 mM en calcium. Les cellules sont traitées deux fois avec les molécules testées: au moment du passage en tampon EDTA puis lors de transfert en tampon Ca²⁺ 3.8mM.

**2 - RESULTATS**
**2.1. Effets sur coeurs isolés de rats soumis à une hypoxie réoxygénation**
Le tableau 1 montre que les composés de l'invention, aux concentrations utilisées: 10⁻⁶M, 3 x 10⁻⁷M ou 5 x 10⁻⁷M, réduisent la contracture se développant après 60 minutes d'hypoxie de 20% à 63% et de 35% à 95% après 30 minutes de réoxygénation. Ils permettent par ailleurs une meilleure récupération fonctionnelle des coeurs au cours de la réoxygénation: la pression ventriculaire des coeurs traités atteint en effet 48% à 89,7% de sa valeur initiale avant hypoxie alors que celle des coeurs contrôles reste limitée de 22.5% à 33,5% de sa valeur initiale.
Le tableau 2 montre que les coeurs provenant de rats traités par le composé de l'exemple 2 par voie orale sont protégés par rapport aux coeurs provenant d'animaux contrôles vis-à-vis des modifications induites par une hypoxie réoxygénation réalisée ex vivo:
1.réduction de la contracture développée après 60 minutes d'hypoxie et 30 minutes de réoxygénation respectivement de 50% et de 37% par rapport à celle développée par les coeurs des animaux contrôles;
2. récupération de la pression ventriculaire (56% de sa valeur initiale) supérieure à celle des coeurs des animaux contrôles (33,5% de sa valeur initiale).

**Tableau 1 :**

| Effet des composés de l'invention sur la contracture et la récupération fonctionnelle de coeurs isolés de rat soumis à une hypoxie réoxygénation. | | | | | |
|---|---|---|---|---|---|
| Composés | Concentration (M) | n | Contracture (mmHg) | | Pression ventriculaire gauche % de la valeur initiale avant hypoxie |
| | | | Hypoxie 60 min | Réoxygénation 30 min | Réoxygénation 30 min |
| Contrôle | | 7 | 48,0±7,1 | 29,3±8,0 | 33,5±8,2 |
| Exemple 1 | 10⁻⁶ | 8 | 32,3±1,6 | 7,8±3,4 | 72,4±9,4 |
| Contrôle | | 12 | 50,2±4,6 | 27,7±4,6 | 22,5±6,2 |
| Exemple 10 | 5x10⁻⁷ | 3 | 18,7±5,8 | 1,3±1,3 | 89,7±11,8 |
| Exemple 15 | 3x10⁻⁷ | 5 | 40,0±6,2 | 18,0±7,2 | 48,1±4,9 |

**Tableau 2 :**

| Effet du composé de l'exemple 2 administré par voie orale chez le rat sur la contracture et la récupération fonctionnelle de coeurs isolés soumis ex vivo à une hypoxie réoxygénation. | | | | |
|---|---|---|---|---|
| Composé | n | Contracture (mmHg) | | Pression ventriculaire gauche % de la valeur initiale avant hypoxie |
| | | Hypoxie 60 min | Réoxygénation 30 min | Réoxygénation 30 min |
| Contrôle | 5 | 47,6±2,4 | 19,2±4,8 | 33,5±8,2 |
| Exemple 2 | 7 | 23,7±6,1 | 12,.0±6,6 | 56,6±7,6 |
| 10mg/kg PO | | | | |

**2.2. - Etudes sur cellules cardiaques**
**2.2.1 - Effets sur la nécrose de cellules cardiaques de rat induite par hypoxie**
   Le tableau 3 montre que les composés de l'invention réduisent la nécrose hypoxique de façon concentration dépendante, dès la concentration de 10⁻⁶M. La protection maximale varie de 49,4 à 76,4% selon les composés. 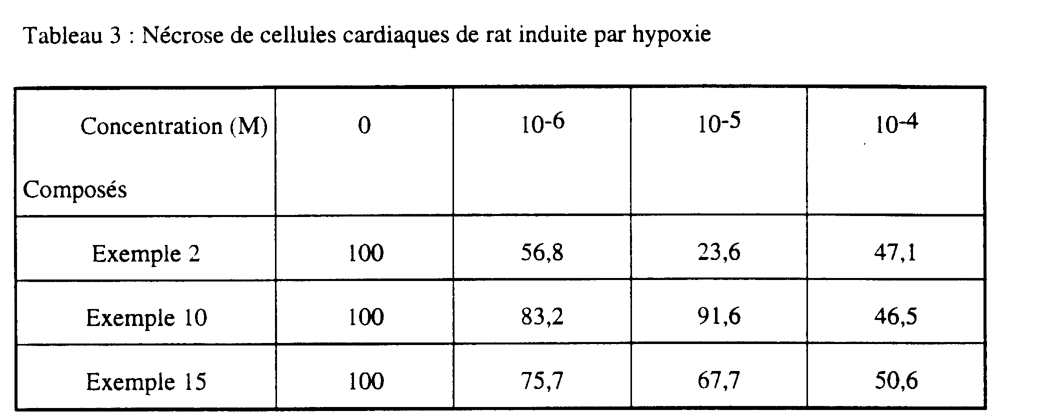 Les résultats sont exprimés en pourcentage par rapport à la nécrose des cellules induites par l'hypoxie seule (index de nécrose 100).
**2.2.2 - Effets sur la nécrose de cellules cardiaques de rat induite par calcium paradoxe**
   Le tableau 4 montre que les composés limitent la nécrose induite par paradoxe du calcium. La protection maximale atteint 70 à 93% aux concentrations de 10⁻⁶M ou 10⁻⁵M. 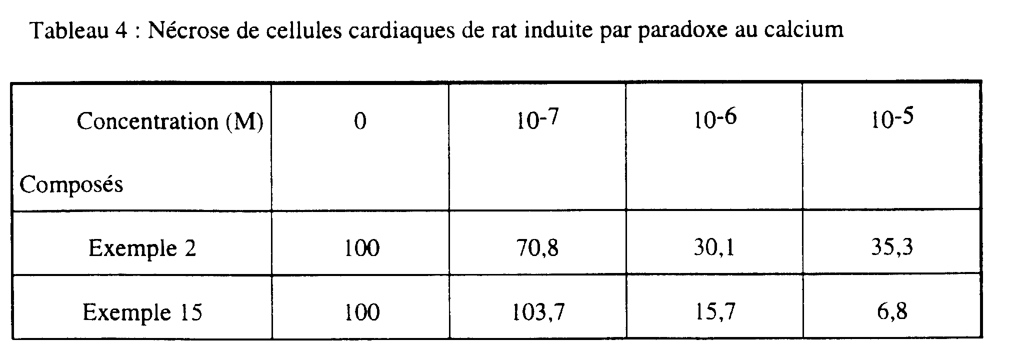 Les résultats sont exprimés en pourcentage par rapport à la nécrose des cellules induites par calcium paradoxe seul (index de nécrose 100).

**B - ETUDE IN VIVO**
**1 - MATERIELS ET METHODES**
L'étude est réalisée sur des porcs "Large White" des deux sexes âgés de 3 mois et pesant 18 à 23 kg.
Les animaux sont anesthésiés au Zoletil® (15mg/kg IM). L'anesthésie est maintenue par une perfusion de 6 à 8 mg/kg/h de thiopental sodique.
Ils sont immédiatement intubés et ventilés avec un mélange air + O₂.
Une thoracotomie en "T" est réalisée par section longitudinale du sternum et incision entre la 4ème et 5ème côte.
Le coeur est suspendu dans un berceau péricardique réalisé par section et fixation du péricarde en quatre points aux muscles thoraciques.
Une bague de débit électromagnétique est placée au niveau de la branche interventriculaire antérieure de la coronaire gauche, un ballonnet pneumatique est placé immédiatement en aval de cette bague. Cet ensemble étant destiné à la réalisation de sténose coronaire sous contrôle de débit. Des cristaux piézo-électriques reliés à un sonomicromètre Triton sont implantés dans le sous endocarde de la paroi ventriculaire gauche selon un plan circonférentiel perpendiculaire à l'axe cardiaque. Ces cristaux sont destinés à l'enregistrement d'ECG endocardiques dans la zone irriguée par l'artère coronaire sténosée.
**2 - PROTOCOLE EXPERIMENTAL**
L'ischémie myocardique est réalisée par inflation du ballonet induisant une réduction du débit coronaire de 50 à 60%.
Deux sténoses identiques de 3 minutes aux effets reproductibles et réversibles sont réalisées, séparées par un intervalle de récupération de 55 minutes.
Le traitement est administré par voie veineuse 10 minutes avant la sténose en perfusion de 5 minutes:
. perfusion de solvant avant la première sténose
. perfusion du produit ou solvant avant la deuxième sténose.

**3 - PARAMETRE ETUDIE**
. La modification des ECG endocardiques en zone ischémique consiste en une surrélévation du segment ST mesurée en millivolt (mV).

**4 - RESULTATS**
Les deux sténoses coronaires réalisées dans le groupe contrôle induisent les mêmes modifications électrocardiographiques. Les composés de l'invention administrés par voie intraveineuse aux doses de 1 ou 3mg/kg avant la seconde sténose coronaire réduisent de 48% à 66% la surrélévation du segment ST des électrocardiogrammes endocardiques par rapport à l'effet de la première sténose coronaire.

| Composés | Surrélévation ST endocardique (mv) | |
|---|---|---|
| | Sténose 1 | Sténose 2 |
| contrôle | 1,9 | 1,9 |
| Exemple 2 1mg/kg | 1,5 | 0,6 |
| Contrôle | 2,8 | 2,9 |
| Exemple 10 3mg/kg | 2,5 | 1,3 |
| Exemple 15 3mg/kg | 1,5 | 0,5 |

## Revendications

1. Les sulfonamides substituées de formule I : 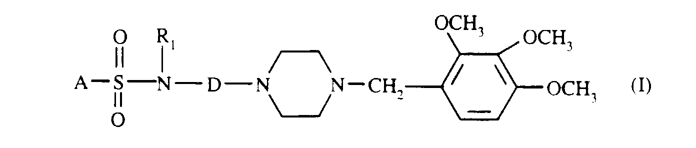 dans laquelle :
- A représente un radical de formule : 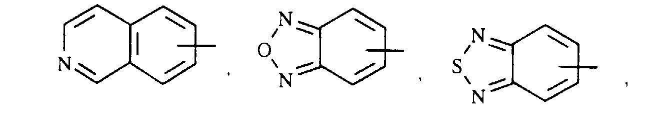 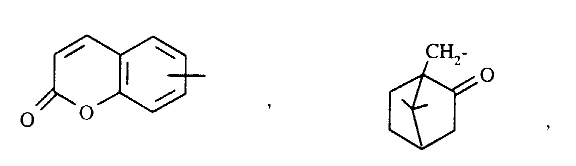 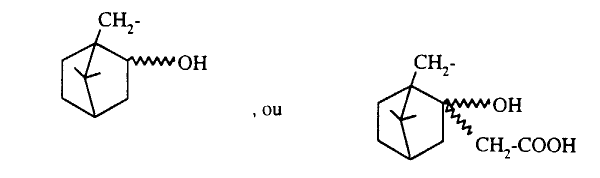
- R₁ représente :
a) un atome d'hydrogène,
b) un radical alkyle linéaire de 1 à 7 atomes de carbone éventuellement substitué par un ou plusieurs radicaux méthyle, phényle, pyridyle ou thiényle, eux-mêmes éventuellement substitués soit par un ou plusieurs atomes d'halogène, soit par un radical hydroxy,
c) un radical alkényle de 3 à 7 atomes de carbone, ou
d) un radical alkynyle de 3 à 7 atomes de carbone ; et
- D représente :
a) une chaîne hydrocarbonée linéaire saturée contenant de 2 à 6 atomes de carbone interrompue par un ou plusieurs atomes d'oxygène, de soufre ou un cycle cyclopropane ou substituée par un gem-diméthyle, ou
b) un groupement de formule : 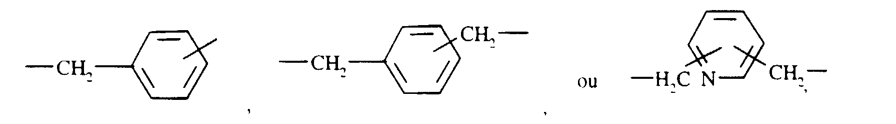
- ainsi que leurs sels d'addition physiologiquement tolérables avec des acides appropriés.

2. Un composé de la revendication 1 qui est N-benzyl N-{3,3-diméthyl 4-[4-(2,3,4-triméthoxybenzyl)pipérazin-1-yl] butyl} (isoquinoléin-5-yl) sulfonamide et son fumarate.

3. Un composé de la revendication 1 qui est le N-(pyrid-3-yl méthyl) N-{3,3-diméthyl 4-[4-(2,3,4-triméthoxybenzyl) pipérazin-1-yl] butyl} (isoquinoléin-5-yl) sulfonamide et son tétrachlorhydrate.

4. Un composé de la revendication 1 qui est le N-éthyl N-{{4-[4-(2,3,4-triméthoxybenzyl)pipérazin- 1-yl]méthyl} benzyl} (isoquinoléin-5-yl)sulfonamide et son trichlorhydrate.

5. Le procédé de préparation des composés de la revendication 1, caractérisé en ce que l'on fait réagir un sulfochlorure de formule II :
A-SO₂ Cl (II)
dans laquelle A prend la signification définie dans la revendication 1,
avec une amine de formule III : 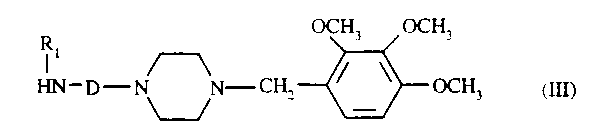 dans laquelle R₁ et D ont les significations définie dans la revendication 1,
et si on le désire, on transforme les dits composés en leurs sels d'addition à un acide pharmaceutiquement acceptable.

6. Le procédé de préparation des composés de la revendication 1 répondant plus précisémment à la formule I' : 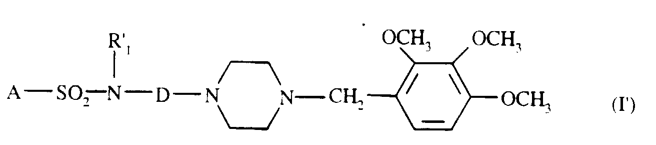 dans laquelle :
A et D ont les significations définies dans la revendication 1 et R'₁ représente :
_ α) un radical alkyle linéaire de 1 à 7 atomes de carbone éventuellement substitué par un ou plusieurs radicaux méthyle, phényle,pyridyle ou thiényle, eux-mêmes éventuellement substitués soit par un ou plusieurs atomes d'halogène, soit par un radical hydroxy,
_ β) un radical alkényle de 3 à 7 atomes de carbone ou
_ δ) un radical alkynyle de 3 à 7 atomes de carbone
caractérisé en ce que l'on fait réagir les composés de formule I'' : 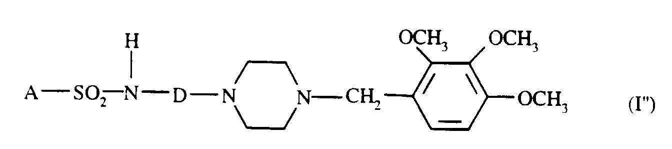 dans laquelle A et D ont les significations définies dans la revendication 1,
avec de l'hydrure de sodium puis un halogénure de formule II' :
R'₁X (II')
dans laquelle R'₁ prend la signification énoncée précédemmment et X représente un atome d'halogène, en opérant dans un solvant approprié
et si on le désire, on transforme les dits composés en leurs sels d'addition à un acide pharmaceutiquement acceptable.

7. A titre de médicaments, un composé selon l'une quelconque des revendications 1 à 4.

8. Les compositions pharmaceutiques, utilisables dans le traitement des pathologies ischémiques myocardiques, de l'accident vasculaire cérébral et des manifestations déficitaires liées aux troubles circulatoires cérébraux chroniques, contenant comme principe actif au moins un des composés selon l'une quelconque des revendications 1 à 4, seul ou en combinaison avec un ou plusieurs excipients pharmaceutiques appropriés.

## Patentansprüche

1. Substituierte Sulfonamide der Formel I: 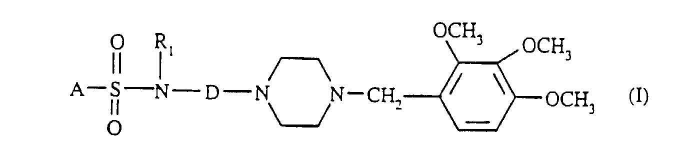 in der:
- A eine Gruppe der Formel: 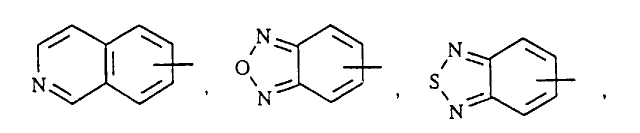 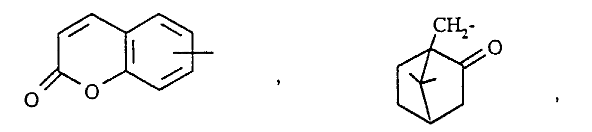 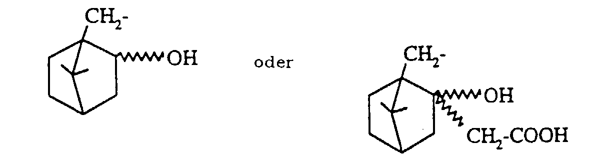
- R₁:
a) ein Wasserstoffatom,
b) eine geradkettige Alkylgruppe mit 1 bis 7 Kohlenstoffatomen, die gegebenenfalls durch eine oder mehrere Methyl-, Phenyl-, Pyridyl- oder Thienylgruppen substituiert ist, die ihrerseits gegebenenfalls entweder durch ein oder mehrere Halogenatome oder durch eine Hydroxygruppe substituiert sind,
c) eine Alkenylgruppe mit 3 bis 7 Kohlenstoffatomen oder
d) eine Alkinylgruppe mit 3 bis 7 Kohlenstoffatomen; und
- D:
a) eine geradkettige gesättigte Kohlenwasserstoffkette mit 2 bis 6 Kohlenstoffatomen, die durch ein oder mehrere Sauerstoffatome, Schwefelatome oder einen Cyclopropanring unterbrochen oder durch eine gem-Dimethylgruppe substituiert ist oder
b) eine Gruppe der Formel: 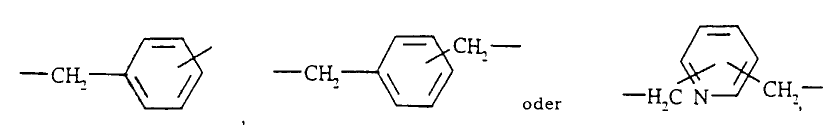 bedeuten
- sowie deren physiologisch verträgliche Additionssalze mit geeigneten Säuren.

2. Verbindung nach Anspruch 1, nämlich N-Benzyl-N-{3,3-dimethyl-4-[4-(2,3,4-trimethoxybenzyl)-piperazin-1-yl]-butyl}-(isochinolin-5-yl)-sulfonamid und dessen Fumarat.

3. Verbindung nach Anspruch 1, nämlich N-(Pyrid-3-yl-methyl)-N-{3,3-dimethyl-4-[4-(2,3,4-trimethoxybenzyl)-piperazin-1-yl]-butyl}-(isochinolin-5-yl)-sulfonamid und dessen Tetrahydrochlorid.

4. Verbindung nach Anspruch 1, nämlich N-Ethyl-N-{{4-[4-(2,3,4-trimethoxybenzyl)-piperazin-1-yl]-methyl}-benzyl}-(isochinolin-5-yl)-sulfonamid und dessen Trihydrochlorid.

5. Verfahren zur Herstellung der Verbindungen nach Anspruch 1, dadurch gekennzeichnet, daß man ein Sulfochlorid der Formel II:
A - SO₂ Cl (II)
in der A die in Anspruch 1 angegebenen Bedeutungen besitzt,
mit einem Amin der Formel III: 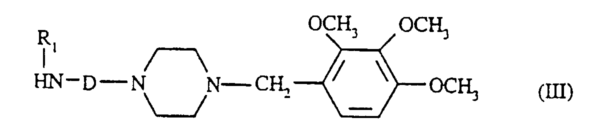 in der R₁ und D die in Anspruch 1 angegebenen Bedeutungen besitzen, umsetzt und gewünschtenfalls die genannten Verbindungen in ihre Additionssalze mit einer pharmazeutisch annehmbaren Säure überführt.

6. Verfahren zur Herstellung der Verbindungen nach Anspruch 1, die genauer der Formel I' entsprechen: 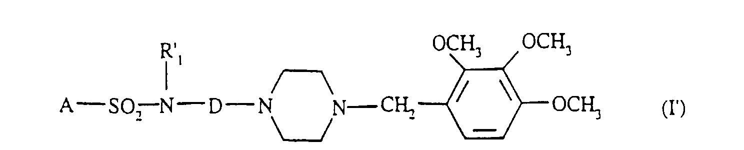 in der: A und D die in Anspruch 1 angegebenen Bedeutungen besitzen und R'₁ :
- α) eine geradkettige Alkylgruppe mit 1 bis 7 Kohlenstoffatomen, die gegebenenfalls durch eine oder mehrere Methyl-, Phenyl-, Pyridyl- oder Thienylgruppen substituiert ist, die ihrerseits gegebenenfalls entweder durch ein oder mehrere Halogenatome oder eine Hydroxygruppe substituiert sind,
- β) eine Alkenylgruppe mit 3 bis 7 Kohlenstoffatomen oder
- δ) eine Alkinylgruppe mit 3 bis 7 Kohlenstoffatomen bedeutet,
dadurch gekennzeichnet, daß man die Verbindungen der Formel I": 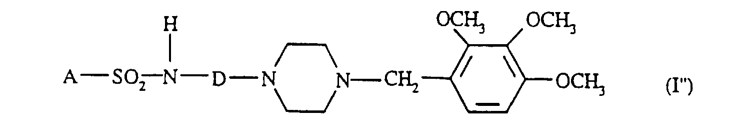 in der A und D die in Anspruch 1 angegebenen Bedeutungen besitzen,
mit Natriumhydrid und dann mit einem Halogenid der Formel II':
R'₁ X (II')
in der R'₁ die oben angegebenen Bedeutungen besitzt und X ein Halogenatom darstellt, umsetzt, wobei man in einem geeigneten Lösungsmittel arbeitet
und gewünschtenfalls die genannten Verbindungen in ihre Additionssalze mit einer pharmazeutisch annehmbaren Säure überführt.

7. Verbindung nach irgendeinem der Ansprüche 1 bis 4 als Arzneimittel.

8. Pharmazeutische Zubereitungen, die zur Behandlung von ischämischen Myokard-Pathologien, zerebralen Gefäßvorfällen und Mangelerscheinungen, die mit chronischen Gehirndurchblutungsstörungen verknüpft sind, geeignet sind, enthaltend als Wirkstoff mindestens eine der Verbindungen nach einem der Ansprüche 1 bis 4 allein oder in Kombination mit einem oder mehreren pharmazeutisch geeigneten Trägermaterialien.

## Claims

1. Substituted sulphonamides of formula I : 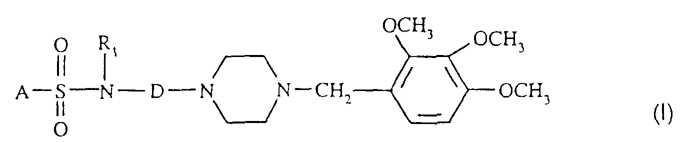 wherein :
- A represents a radical of formula 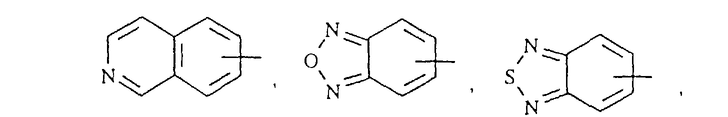 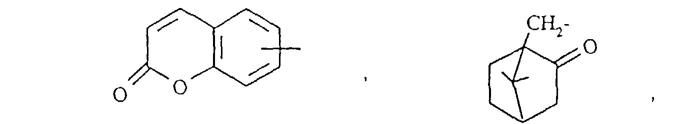 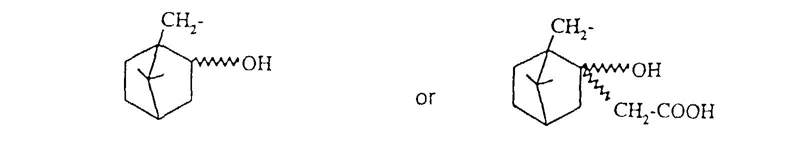
- R₁ represents :
a) a hydrogen atom,
b) a linear alkyl radical having from 1 to 7 carbon atoms optionally substituted by one or more methyl, phenyl, pyridyl or thienyl radicals which are themselves optionally substituted either by one or more halogen atoms or by a hydroxy radical,
c) an alkenyl radical having from 3 to 7 carbon atoms, or
d) an alkynyl radical having from 3 to 7 carbon atoms; and
- D represents
a) a saturated linear hydrocarbon chain containing from 2 to 6 carbon atoms interrupted by one or more oxygen atoms or sulphur atoms or by a cyclopropane ring or substituted by a gem-dimethyl, or
b) a group of formula : 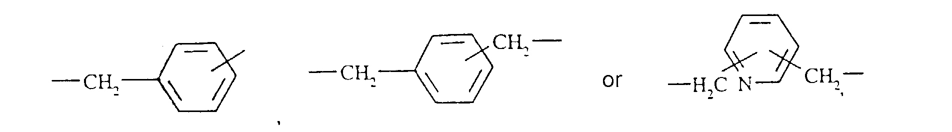
- and also physiologically tolerable addition salts thereof with appropriate acids.

2. A compound of claim 1, which is N-benzyl-N-{3,3-dimethyl-4-[4-(2,3,4-trimethoxybenzyl)piperazin -1-yl]butyl}-(isoquinolin-5-yl)sulphonamide and its fumarate.

3. A compound of claim 1, which is N-(pyrid-3-ylmethyl)-N-{3,3-dimethyl-4-[4-(2,3,4-trimethoxybenzyl)piperazin-1-yl]butyl}-(isoquinolin-5-yl)sulphonamide and its tetrahydrochloride.

4. A compound of claim 1, which is N-ethyl-N-{{4-[4-(2,3,4-trimethoxybenzyl)piperazin-1-yl]methyl}benzyl}-(isoquinolin-5-yl)sulphonamide and its trihydrochloride.

5. A process for the preparation of the compounds of claim 1, which is characterised in that a sulphochloride of formula II :
A-SO₂Cl (II),
wherein A is as defined in claim 1, is reacted with an amine of formula III : 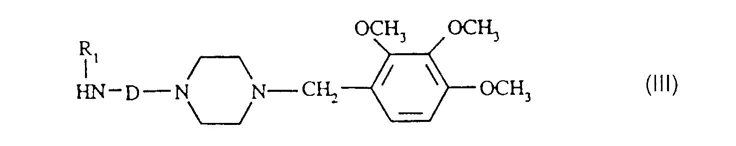 wherein R₁ and D are as defined in claim 1,
and, if desired, the said compounds are converted into addition salts with a pharmaceutically acceptable acid.

6. A process for the preparation of the compounds of claim 1 corresponding more specifically to formula I' : 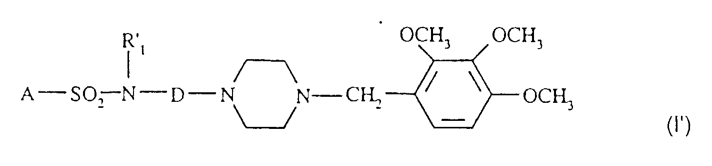 wherein : A and D are as defined in claim 1 and R'₁ represents :
- α) a linear alkyl radical having from 1 to 7 carbon atoms optionally substituted by one or more methyl, phenyl, pyridyl or thienyl radicals which are themselves optionally substituted either by one or more halogen atoms or by a hydroxy radical,
- β) an alkenyl radical having from 3 to 7 carbon atoms or
- δ) an alkynyl radical having from 3 to 7 carbon atoms,
characterised in that the compounds of formula I" : 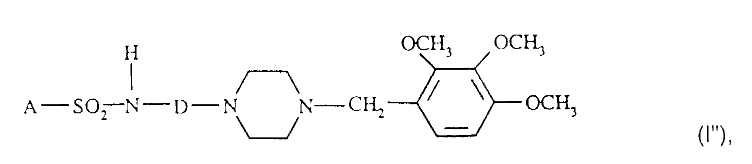 wherein A and D are as defined in claim 1, are reacted with sodium hydride and then with a halide of formula II' :
R'₁X (II')
wherein R'₁ is as defined above and X represents a halogen atom, the reaction being carried out in an appropriate solvent,
and, if desired, the said compounds are converted into addition salts with a pharmaceutically acceptable acid.

7. As medicaments, a compound according to any one of claims 1 to 4.

8. Pharmaceutical compositions, which can be used in the treatment of myocardial ischaemic pathologies, cerebral vascular accident and manifestations of deficiencies associated with chronic cerebral circulatory disorders, comprising as active ingredient at least one compound according to any one of claims 1 to 4, alone or in combination with one or more pharmaceutically appropriate excipients.
